# EUROPEAN PATENT APPLICATION

(11) **EP 4 729 625 A1**
(43) Date of publication of application: **22.04.2026**
(21) Application number: 24868696.6
(22) Date of filing: 20.09.2024
(51) Int. Cl.: C12P 13/12, C07C 319/28, C07C 323/58

(54) **METHOD FOR CONTROLLING CHROMATICITY OF NATURAL L-CYSTEINE HYDROCHLORIDE CRYSTALS**

(30) Priority: 21.09.2023 KR 20230126192
(71) Applicant: CJ Cheiljedang Corporation, Seoul 04560 (KR)
(72) Inventor: KIM, Jungwon, Seoul 04560 (KR); LEE, Kang Hoon, Seoul 04560 (KR); HONG, Je-Won, Seoul 04560 (KR); PARK, Shin-Ae, Seoul 04560 (KR); YOO, Hyun-Woo, Seoul 04560 (KR)
(74) Representative: Meissner Bolte Partnerschaft mbB
(86) International application number: PCT/KR2024/014146
(87) International publication number: WO 2025/063719

(57) **Abstract**

The present invention relates to a method for producing L-cysteine hydrochloride crystals with reduced yellowness from an L-cysteine process liquid derived from a fermentation liquid. The method for producing L-cysteine crystals comprises the steps of: (a) obtaining an L-cysteine separation solution from a fermentation solution containing L-cysteine; (b) filtering the L-cysteine separation solution to obtain a filtrate; (c) concentrating the filtrate to obtain a concentrate; (d) adding hydrochloric acid to the filtrate or concentrate before or after step (c); and (e) cooling the concentrate to recover L-cysteine crystals.

## Description

### [TECHNICAL FIELD]

### Cross-Reference to Related Application(s)

The present application claims the benefit of priority based on Korean Patent Application No. 10-2023-0126192, filed on September 21, 2023, and all contents disclosed in the documents of the said Korean Patent Application is incorporated herein by reference.

The present invention relates to a method for controlling the chromaticity of natural L-cysteine hydrochloride crystals.

### [BACKGROUND ART]

L-cysteine is an amino acid that plays an important role in sulfur metabolism in all organisms. It is used in the biosynthesis of proteins such as hair keratin, glutathione, biotin, methionine, and other sulfur-containing metabolites, as well as serving as a precursor for Coenzyme A. Furthermore, L-cysteine and its derivatives can be used in various industrial fields, including the pharmaceutical industry (for treating bronchial diseases), the cosmetics industry (hair shampoos, compositions for permanent waves), and the food industry (antioxidants, flavor enhancers).

L-cysteine is generally prepared by decomposing animal-derived L-cystine which is sourced from raw materials such as duck feathers or human hair, or fermentation-derived L-cystine which is sourced from microbial culture fluid, into L-cysteine using an electrochemical reduction reaction. In contrast, the known methods for producing L-cysteine using microorganisms include a fermentation process that produces natural L-cysteine using a strain modified with O-acetyltransferase in a sulfur-containing medium (US 8802399 B), and a process for producing natural L-cysteine by mixing O-phosphohomoserine produced by a microbial culture method with sulfide and inducing an enzyme-catalyzed reaction using O-phosphoserine sulfhydrylase (WO 2013-089478 A3). Furthermore, a process for producing L-cysteine hydrochloride hydrate (WO 2019-151769 A1) is known, which separates and purifies L-cysteine fermentation broth in its natural state using a continuous chromatography process without chemical reactions or the use of artificial synthetic compounds, offering advantages such as high yield and purity, enhanced production efficiency, and reduced water consumption.

However, when using a continuous chromatography process, separation of the final product from coloring substances originating from the fermentation medium or fermentation is required, and an additional purification step may be necessary. Although activated carbon is mainly used to remove the coloring substances, in the case of fermentation broth produced from L-cysteine fermentation fluid using the aforementioned method, coloring substances and impurities that are not removed by activated carbon may develop color under the conditions for producing cysteine hydrochloride crystals, resulting in coloration of the final product and deterioration of color quality.

Under the background, an objective of the present invention is to provide a method for preparing L-cysteine hydrochloride crystals with uniform chromaticity from L-cysteine fermentation broth produced by microbial culturing method and enzyme-catalyzed reactions.

### [DISCLOSURE]

### [TECHNICAL PROBLEM]

An embodiment of the present application provides a method for preparing L-cysteine crystals, comprising steps of:
(a) obtaining an L-cysteine separated solution from a fermentation broth comprising L-cysteine;
(b) filtering the L-cysteine separated solution to obtain a filtrate;
(c) concentrating the filtrate to obtain a concentrate;
(d) adding hydrochloric acid to the filtrate or the concentrate before or after step (c); and
(e) cooling the concentrate to recover L-cysteine crystals.

Another embodiment of the present application provides L-cysteine crystals characterized by a yellowness of 4.0 or less.

### [TECHNICAL SOLUTION]

Hereinafter, the present invention will be described in more detail.

Each of the explanations and exemplary embodiments disclosed herein can be applied to other explanations and exemplary embodiments. That is, all combinations of various factors disclosed herein belong to the scope of the present disclosure. Furthermore, the scope of the present disclosure should not be limited by the specific disclosure provided hereinbelow.

In one aspect, the present application provides a method for preparing L-cysteine crystals, comprising:
(a) obtaining an L-cysteine separated solution from a fermentation broth comprising L-cysteine;
(b) filtering the L-cysteine separated solution to obtain a filtrate;
(c) concentrating the filtrate to obtain a concentrate;
(d) adding hydrochloric acid to the filtrate or concentrate before or after step (c); and
(e) cooling the concentrate to recover L-cysteine crystals.

The method for preparing L-cysteine crystals of the present application is described in detail step by step as follows.

### (a) Step: Obtaining an L-cysteine separated solution

The method for preparing L-cysteine crystals of the present application may include step (a) of obtaining an L-cysteine separated solution from a fermentation broth comprising L-cysteine.

As used herein, the term "L-cysteine" refers to an amino acid that constitutes proteins and, among L-amino acids, is the only sulfur-containing amino acid with a thiol group (R-SH). L-cysteine may be chemically synthesized or biologically produced, such as through microbial fermentation, but is not limited thereto. Specifically, the L-cysteine in the present application may be L-cysteine biologically produced through microbial fermentation, or may be natural L-cysteine obtained by inducing an enzymatically catalyzed reaction between O-phosphohomoserine of a precursor produced through microbial fermentation, and a sulfide in the presence of phosphoserine sulfhydrylase. The natural L-cysteine may be L-cysteine that has not undergone a chemical reaction, chemical adsorption, or elution step during the manufacturing process.

In present application, the term "natural" indicates that something does not depend on a chemical reaction. According to the EU Flavor Regulation No. 1334/2008, only substances obtained by a physical, enzymatic, or microbial process are defined as "natural" flavoring agents. From the above viewpoint, regardless of whether it is derived from an animal or microbial fermentation, L-cysteine produced by an electrochemical reduction reaction of L-cystine cannot be called entirely natural.

In the present application, the term "fermentation broth" refers to a culture broth obtained by culturing a microorganism that produces L-cysteine, a culture comprising a microorganism cultured with the medium, or an enzymatic conversion broth comprising precursors and enzymes capable of producing L-cysteine. Specifically, the fermentation broth comprising L-cysteine may be a culture broth or an enzymatic conversion broth comprising natural L-cysteine. More specifically, it may be an L-cysteine culture or culture solution biologically produced through the fermentation of a microbe capable of producing L-cysteine. Alternatively, it may be an enzymatically converted solution of natural L-cysteine obtained by inducing an enzyme-catalyzed reaction between O-phosphohomoserine of a precursor produced through microbial fermentation, and a sulfide in the presence of phosphoserine sulfhydrylase. L-cysteine crystals produced using the fermentation solution as a raw material solution are not produced by a chemical reaction and thus, can be considered natural L-cysteine.

In the present application, the step of obtaining the separated solution in step (a) may be performed using continuous chromatography. In one example, the fermentation broth may be used as a raw material solution for the continuous chromatography process. That is, it may be injected into the continuous chromatography device in step (a).

The pH of the fermentation broth injected into the continuous chromatography device may vary depending on the preparation method, but may be a pH of 6.0 to 11.5, 6.5 to 11.0, 7.0 to 10.5, 7.5 to 10.0, or 8.0 to 9.5. The fermentation broth itself may be used as a raw material solution for the continuous chromatography process, and a step of adjusting the pH of the fermentation broth comprising L-cysteine to 6.0 to 11.5, 6.5 to 11.0, 7.0 to 10.5, 7.5 to 10.0, or 8.0 to 9.5 prior to step (a) may additionally be included. For example, the adjustment may be affected by adding an acid such as sulfuric acid or hydrochloric acid, or a base such as sodium hydroxide (caustic soda), ammonia, lithium hydroxide, or potassium hydroxide, but is not limited thereto. The pH adjusting agent can be appropriately selected and used by a person skilled in the art as long as it does not affect the structure of L-cysteine and can ultimately obtain crystals of L-cysteine.

The term "continuous chromatography" used in the present application refers to a continuous process that is evolved from the conventional batch chromatography process. Specifically, solid and liquid phases can be continuously supplied to the chromatography device, and the solid and liquid phases move in opposite directions to cause countercurrent contact, thereby enabling more efficient separation of substances. In present application, the term "continuous chromatography" includes both true moving bed (TMB) chromatography and simulated moving bed (SMB) chromatography. Furthermore, since both true moving bed and simulated moving bed chromatography share the same principles, those skilled in the art can select and use them appropriately, by considering productivity and other factors.

The use of continuous chromatography process in present application eliminates the need for an adsorption/elution step, thereby offering the advantages of higher productivity per hour and reduced process water usage compared to ion exchange processes. Furthermore, to obtain a high yield of L-cysteine powder from a process solution comprising L-cysteine obtained through an ion exchange process or a general chromatography process, a significant amount of energy is required for the concentration and crystallization process. However, the method of the present application can reduce energy costs.

The stationary phase of the continuous chromatography device may be an ion exchange resin, specifically a strongly acidic cation exchange resin. The functional group of the strongly acidic cation exchange resin may be a sulfate group, but is not limited thereto. Furthermore, the core material of the strongly acidic cation exchange resin used herein is not limited as long as it can have a strongly acidic functional group attached to it. For example, a styrene-divinylbenzene copolymer may be used, but is not limited thereto. For example, the strongly acidic cation exchange resin may be styrene-divinylbenzene copolymer, but not limited thereto. For a specific example, a sulfated styrene-divinylbenzene copolymer may be used, but is not limited thereto.

In the present application, it is difficult to purify the solid content of L-cysteine in the separated solution obtained through a continuous chromatography process to 50% or more (w/w) by using other types of stationary phases frequently used in the art for separation and purification of amino acid, such as non-functionalized exchange resins such as non-functionalized styrene-divinylbenzene copolymers and non-functionalized methacrylate polymers; strongly basic anion exchange resins such as trimethylamine styrene-divinylbenzene copolymers; weakly basic anion exchange resins such as tertiary amine styrene-divinylbenzene copolymers; and weakly basic cation exchange resins such as carboxyl methacrylate polymers. On the other hand, when using a strongly acidic cation exchange resin such as a sulfated styrene-divinylbenzene copolymer, the solid content excluding water of L-cysteine obtained through a continuous chromatography process can be purified to at least 80% (w/w), specifically, at least 90% (w/w).

The chromatography device can use water, diluted caustic soda, diluted sulfuric acid, diluted phosphoric acid, diluted hydrochloric acid, diluted potassium hydroxide, or mixtures thereof as the mobile phase without added synthetic chemical compounds (e.g., organic solvents such as methanol, isopropyl alcohol, or acetonitrile), but is not limited thereto. For specific example, water can be used as the mobile phase for continuous chromatography. When using a mobile phase containing synthetic chemical compounds, the synthetic chemical compounds may remain in the final product to potentially exceed the residue standard for chemical compounds, so that it may be prevented from selling or distributing as a natural product. Furthermore, since no chemical compounds other than water are added to the process water, a cost reduction effect can be expected. In the ion exchange resin process, solvents such as hydrochloric acid or sulfuric acid must be used as eluents to elute the adsorbed L-cysteine, which inevitably increases product costs related to the use and disposal of these chemicals.

In step (a), the fermentation broth comprising L-cysteine is injected into a continuous chromatography device. The L-cysteine is separated through the continuous chromatography process, thereby obtaining a separated solution comprising L-cysteine. In the present application, the separated solution comprising L-cysteine may be referred to simply as "separated solution," "chromatographic separated solution," or "process solution."

### (b) Step: Obtaining an L-cysteine filtrate

Thereafter, the method of the present application may include step (b) of filtering the L-cysteine separated solution obtained in step (a) to obtain a filtrate.

In step (b), any method known in the art may be used to filter the L-cysteine separated solution. For example, a filtration method using a Nutsche filter, a filter press, or a housing filter using a filter made of CA, PVDF, PES, or PTFE may be used. In one embodiment, a Nutsche filter may be used for filtration, but is not limited thereto.

The (b) step of obtaining filtrate may be performed by adding activated carbon. In one example, the activated carbon can be added in an amount of 0.01 to 50 wt%, 0.1 to 50 wt%, 1 to 50 wt%, 5 to 50 wt%, 0.01 to 40 wt%, 0.1 to 40 wt%, 1 to 40 wt%, 5 to 40 wt%, 0.01 to 30 wt%, 0.1 to 30 wt%, 1 to 30 wt%, 5 to 30 wt%, 0.01 to 20 wt%, 0.1 to 20 wt%, 1 to 20 wt%, 2 to 20 wt%, 3 to 20 wt%, 4 to 20 wt%, 5 to 20 wt%, 6 to 20 wt%, 7 to 20 wt%, 8 to 20 wt%, 9 to 20 wt%, 0.01 to 15 wt%, 0.1 to 15 wt%, 1 to 15 wt%, 2 to 15 wt%, 3 to 15 wt%, 4 to 15 wt%, 5 to 15 wt%, 6 to 15 wt%, 7 to 15 wt%, 8 to 15 wt%, 9 to 15 wt%, 0.01 to 12 wt%, 0.1 to 12 wt%, 1 to 12 wt%, 2 to 12 wt%, 3 to 12 wt%, 4 to 12 wt%, 5 to 12 wt%, 6 to 12 wt%, 7 to 12 wt%, 8 to 12 wt%, or 9 to 12 wt% based on total weight of L-cysteine, but is not limited thereto.

### (c) Step: Obtaining an L-cysteine concentrate

Thereafter, the method of the present application may include step (c) of concentrating the L-cysteine filtrate obtained in step (b) to obtain a concentrate.

The concentration in step (c) may be performed in a conventional concentrator, such as a forced circulation concentrator, a thin-film concentrator, or a rotary concentrator. In one example, the concentration may be performed in a rotary concentrator.

The vacuum of the concentrator in the concentration step (c) may be 90 to 130 mmHg, 95 to 125 mmHg, or 100 to 120 mmHg, such as 110 mmHg, but is not limited to. Furthermore, the external temperature of the concentrator may be, 50 to 90°C, 55 to 85°C, or 60 to 80°C, for example 70°C, but is not limited to.

### Step (d): Adding hydrochloric acid

The method of the present application may include a step (d) of adding hydrochloric acid to the filtrate or concentrate, before or after step (c).

The amount of hydrochloric acid added in step (d) may be such that the hydrochloric acid concentration of the concentrate after addition is less than 6.5, less than 6.0 N, less than 5.5 N, less than 4.5 N, between 4.5 N or higher and less than 5.5 N, or between 5.5N or higher and less than 6.5 N, but is not limited thereto.

The hydrochloric acid addition step in step (d) may be performed before step (e), after step (b), or specifically, before step (c), after step (c), or simultaneously with step (c). The method for preparing L-cysteine crystals of the present application may further includes a step of controlling the residence time of the concentrate at a temperature of 10°C to 70°C, after step (d).

The term "residence time" of the concentrate in the present application may refer to the time for which the concentrate is maintained in a liquid state before crystallization, and may be used interchangeably with the terms "storage time," "concentration time," "maintaining time," or "holding time."

In the present application, the residence time of the concentrate may vary depending on the hydrochloric acid concentration and residence temperature of the concentrate after the addition of hydrochloric acid in step (d).

In one example,
the residence time of the concentrate at 10°C or higher and less than 30°C of the residence temperature may be
   (i) within 24 hours, when the hydrochloric acid concentration of the concentrate after the addition of hydrochloric acid in step (d) is 4.5 N or higher;
the residence time of the concentrate at 30°C or higher and less than 50°C of the residence temperature may be
   (i) within 720 minutes, when the hydrochloric acid concentration of the concentrate after the addition of hydrochloric acid in step (d) is less than 4.5 N,
   (ii) within 480 minutes, when the hydrochloric acid concentration of the concentrate after the addition of hydrochloric acid in step (d) is between 4.5 N or higher and less than 5.5 N, and/or
   (iii) within 240 minutes, when the hydrochloric acid concentration of the concentrate after the addition of hydrochloric acid in step (d) is between 5.5 N or higher and less than 6.5 N;
the residence time of the concentrate at 50°C or higher of the residence temperature may be
   (i) within 240 minutes, when the hydrochloric acid concentration of the concentrate after the addition of hydrochloric acid in step (d) is less than 4.5 N,
   (ii) within 180 minutes, if the hydrochloric acid concentration of the concentrate after the addition of hydrochloric acid in step (d) is between 4.5 N or higher and less than 5.5 N, and/or
   (iii) within 120 minutes, when the hydrochloric acid concentration of the concentrate after the addition of hydrochloric acid in step (d) is 5.5 N or higher and less than 6.5 N, but is not limited thereto.

Specifically, the residence time of the concentrate at 15 °C to 25°C of the residence temperature may be
(i) within 540 minutes, when the hydrochloric acid concentration of the concentrate after the addition of hydrochloric acid in step (d) is 4.5 N or higher;

the residence time of the concentrate at 35°C to 45°C of the residence temperature may be,
   (i) within 540 minutes, when the hydrochloric acid concentration of the concentrate after the addition of hydrochloric acid in step (d) is less than 4.5 N,
   (ii) within 420 minutes, when the hydrochloric acid concentration of the concentrate after the addition of hydrochloric acid in step (d) is 4.5 N or higher and less than 5.5 N, and/or
   (iii) within 210 minutes, when the hydrochloric acid concentration of the concentrate after the addition of hydrochloric acid in step (d) is 15.5 N or higher and less than 6.5 N;
the residence time of the concentrate at 55°C to 65°C of the residence temperature may be
   (i) within 210 minutes, when the hydrochloric acid concentration of the concentrate after the addition of hydrochloric acid in step (d) is less than 4.5 N;
   (ii) within 150 minutes, when the hydrochloric acid concentration of the concentrate after the addition of hydrochloric acid in step (d) is 4.5 N or higher and less than 5.5 N, and/or
   (iii) within 90 minutes, when the hydrochloric acid concentration of the concentrate after the addition of hydrochloric acid in step (d) is 5.5 N or higher and less than 6.5 N, but is not limited thereto.

In the present application, the residence temperature of the concentrate may refer to the temperature at which the concentrate is maintained constantly during the residence time, and may refer to the temperature of the concentrate.

The residence temperature of the concentrate may be 10°C, 15°C or higher, 20°C or higher, 25°C or higher, 30°C or higher, 40°C or higher, 45°C or higher, 50°C or higher, 55°C or higher, 10 to 70°C, 15 to 70°C, 20 to 70°C, 25 to 70°C, 30 to 70°C, 35 to 70°C, 40 to 70°C, 45 to 70°C, 50 to 70°C, 55 to 70°C, 10 to 65°C, 15 to 65°C, 20 to 65°C, 25 to 65°C, 30 to 65°C, 35 to 65°C, 40 to 65°C, 45 to 65°C, 50 to 65°C, 10°C or higher and less than 30°C, 15°C to 25°C, 30°C or higher and less than 50°C, 35°C to 45°C, or 55°C to 65°C.

After adjusting the residence time of the concentrate, the absorbance at 430 nm of the L-cysteine concentrate may be 0.65 or less, 0.60 or less, or 0.55 or less, but is not limited thereto.

The L-cysteine concentrate after adjusting the residence time of the concentrate may exhibit a relatively low absorbance value due to reduced reddening compared to an L-cysteine concentrate that has not undergone the above process, by adding hydrochloric acid before or after the concentration step, and/or adjusting the residence time according to the hydrochloric acid concentration of the concentrate after adding hydrochloric acid.

In the present application, the L-cysteine concentrate, which does not undergo the step of adding hydrochloric acid before or after the concentration step, and/or the step of adjusting the residence time according to the hydrochloric acid concentration of the concentrate after the addition of hydrochloric acid, may comprise a "color-inducing substance" that cause the reddening phenomenon. The method of the present application includes a step for reducing the "color-inducing substance," which includes the step of adding hydrochloric acid before or after the concentration step and/or the step of adjusting the residence time according to the hydrochloric acid concentration of the concentrate after the addition of hydrochloric acid.

The term "color-inducing substance" in the present application refers to a color-inducing substance, a substance that causes the reddening phenomenon, and may be used interchangeably with the terms "color-causing substance" or "color-generating substance."

In the present application, the color-inducing substances included in the L-cysteine concentrate that causes the reddening phenomenon may be PLP (pyridoxal-5'-phosphate), but is not limited to.

### (e) Step: Cooling the concentrate and recovering the crystals

Thereafter, the method of the present application may include step (e) of cooling the concentrate added by hydrochloric acid, to recover L-cysteine crystals.

The method may further include a step of crystallizing L-cysteine after the cooling in step (e).

The term "crystallization" as used herein refers to a phenomenon in which a liquid or amorphous phase forms crystals, which occurs through two phenomena: the generation of crystal nuclei and the growth of crystal nuclei.

The concentrate may be cooled and/or aged prior to recovery to induce the formation and/or growth of crystal nuclei. Furthermore, even if L-cysteine crystals do not precipitate in the concentrate, crystals may form when the concentrate is cooled and/or aged. The cooling step may specifically refer to cooling to a temperature of -10 to 55°C for a period of 2 to 6 hours, specifically to a temperature of 0 to 45°C for a period of 2 to 6 hours, more specifically to a temperature of 0 to 30°C for a period of 2 to 6 hours, and even more specifically to a temperature of 15°C for a period of 2 to 6 hours.

The aging step may refer to leaving the solution without temperature change. In the present application, it may also refer to maintaining the cooled temperature at a constant level, and even when cooling is not performed, it may refer to maintaining the temperature of the concentrate at a constant level. Specifically, the aging may be performed for a period of 1 to 3 hours.

In step (e), the L-cysteine crystals precipitated from the concentrate may be recovered. Specifically, the concentrate may be subjected to solid-liquid separation to recover L-cysteine crystals from the slurry. This can be accomplished using a solid-liquid separator, such as a vacuum membrane filtration device, a pressurized membrane filtration device, or a centrifugal separator, but is not limited thereto. The slurry and/or the precipitated L-cysteine crystals may additionally undergo a washing or drying step.

In the present application, the L-cysteine crystals produced through steps (a) to (e) can maintain uniform yellowness.

The yellowness of the L-cysteine crystals is one of the chromaticity measured by a color difference meter, and may be used interchangeably with the b or b* value in the present application.

The yellowness of the L-cysteine crystals may be less than 7.1, 7.0 or less, 6s.5 or less, 5.0 or less, 4.5 or less, 4.0 or less, 1.5 to 7.0, 1.5 to 6.5, 1.5 to 5.0, 1.5 to 4.5, 1.5 to 4.0, 2.0 to 7.0, 2.0 to 6.5, 2.0 to 5.0, 2.0 to 4.5, 2.0 to 4.0, 2.1 to 7.0, 2.1 to 6.5, 2.1 to 5.0, 2.1 to 4.5, 2.1 to 4.0, 2.5 to 7.0, 2.5 to 6.5, 2.5 to 5.0, 2.5 to 4.5, or 2.5 to 4.0.

The term " color difference meter" in present application refers to a device for measuring the color of an object. The color difference meter used for color measurement in present application may be, but is not limited to, a CHROMA METER (CR-410, KONICA MINOLTA).

In present application, the L-cysteine concentrate after controlling the residence time of the L-cysteine concentrate, the L-cysteine crystals prepared by adding hydrochloric acid before or after the concentration step and/or adjusting the residence time according to the hydrochloric acid concentration of the concentrate after adding hydrochloric acid, exhibit relatively low yellowness due to reduced reddening, compared to L-cysteine crystals that have not undergone the above process.

In the present application, L-cysteine crystals that do not undergo the step of adding hydrochloric acid before or after the concentration step and/or the step of adjusting the residence time according to the hydrochloric acid concentration of the concentrate after the addition of hydrochloric acid, may comprise a "color-inducing substance" that causes reddening phenomenon. The method of the present application includes a step of reducing the "color-inducing substance," which includes the step of adding hydrochloric acid before or after the concentration step, and/or the step of adjusting the residence time according to the hydrochloric acid concentration of the concentrate after the addition of hydrochloric acid.

In the present application, the color-inducing substance comprised in the L-cysteine concentrate that causes reddening phenomenon may be PLP (pyridoxal-5'-phosphate), but is not limited to.

In another aspect, the present application provides L-cysteine crystals characterized by a yellowness of 4.0 or less.

The L-cysteine crystals are prepared by a method for preparing L-cysteine crystals, comprising
(a) obtaining an L-cysteine separated solution from a fermentation broth containing L-cysteine;
(b) filtering the L-cysteine separated solution to obtain a filtrate;
(c) concentrating the filtrate to obtain a concentrate;
(d) adding hydrochloric acid to the filtrate or concentrate before or after step (c); and
(e) cooling the concentrate added with hydrochloric acid, to recover L-cysteine crystals.

The method for preparing L-cysteine crystals is as described above.

### [BRIEF DESCRIPTION OF THE DRAWING]

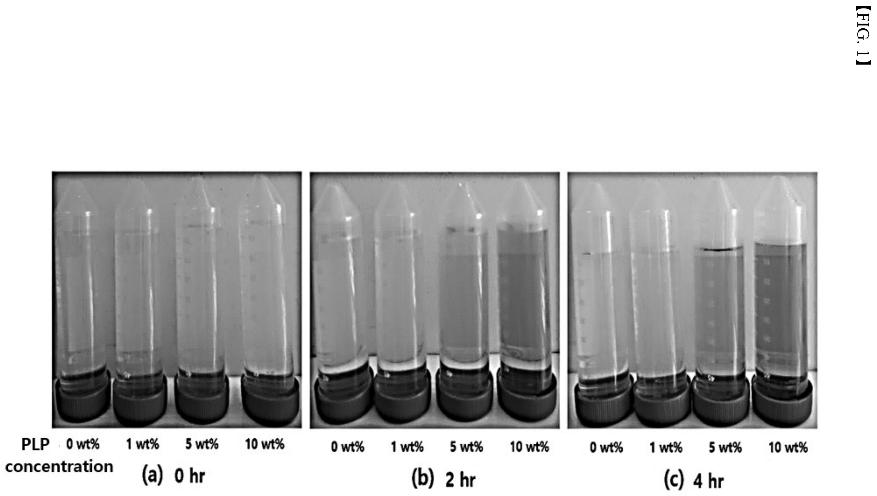

Fig. 1 is a drawing showing color change of L-cysteine solutions according to the concentration of pyridoxal 5'-phosphate (PLP) added and residence time: the color of L-cysteine solutions (a) at 0 h after PLP addition, (b) at 2 h after PLP addition, and (c) at 4 h after PLP addition.

### [MODE FOR INVENTION]

The present invention will be described in more detail below through examples. However, these examples are intended to illustrate one or more specific examples and are not intended to limit the scope of the present invention to these examples.

### Example 1. Comparison of color intensity of fermentation-derived hydrochloride crystals containing L-cysteine according to hydrochloric acid concentration

### Example 1-1. Production of fermentation-derived process solution containing L-cysteine using microbial fermentation

O-phosphohomoserine of a precursor produced through microbial fermentation and sulfide were subjected to an enzymatically catalyzed reaction using O-phosphoserine sulfhydrase (OPS sulfhydrylase) and pyridoxal 5'-phosphate (PLP), to produce a natural L-cysteine fermentation solution. The pH of the L-cysteine fermentation solution ranged from 8 to 9.5. Specifically, KCCM 11103P strain (CA07-0022/pCL-prmf-serA*(G336V)-serC; Korea Patent No. 10-1381048) which has OPS production ability due to the deletion of serB and the introduction of a mutant serA* in the E. coli W3110 strain, was cultured on an MMYE agar plate at 33°C for 24 hours, and 1/10 of the cells on the plate were scraped from one plate and seeded in a baffle flask with flask seed medium (10 g/L glucose, 0.5 g/L magnesium sulfate, 3 g/L potassium dihydrogen phosphate, 10 g/L yeast extract, 0.5 g/L sodium chloride, 1.5 g/L ammonium chloride, 12.8 g/L sodium pyrophosphate, 1 g/L glycine) and performed for seed culture at 30°C at 200 rpm for 6 hours. After completion of seed culture, a volume of seed culture medium corresponding to 16% of the main culture medium volume was inoculated into a 1 L small fermenter filled with 300 mL of main culture medium, and cultured at 33°C at pH 7.0 to obtain an OPS fermentation broth. The 50 mM OPS fermentation broth was reacted with 50 mg/ml Msm-T enzyme from Mycobacterium tuberculosis H37Rv in the presence of 100 mM Na₂S and 0.2 mM pyridoxal 5'-phosphate (PLP) to obtain a fermentation broth containing L-cysteine (US 8557549 B2).

The obtained L-cysteine fermentation broth was filtered through a 0.14 um membrane to obtain a filtrate solution and separate microbial cells. The pH of the filtrate solution was adjusted down to pH 2-7 using 98% sulfuric acid. Then, a continuous chromatography using a strongly acidic cation exchange resin process (US 11427537 B2) was used to separate L-cysteine of a separated solution.

10 wt% of activated carbon relative to the total weight of L-cysteine was added to the L-cysteine separated solution and stirred at 40°C for 1 hour. The activated carbon was separated using a Nutsche filter to obtain a filtrate solution.

### Example 1-2. Comparison of color intensity of L-cysteine according to hydrochloric acid concentration

The following experiment was conducted to compare the color differences between the L-cysteine concentrate and the L-cysteine hydrochloride crystals recovered by cooling the concentrate, when hydrochloric acid was added to the filtrate obtained by separation using a Nutsche filter in Example 1-1.

Specifically, the filtrate obtained in Example 1-1 was concentrated in a rotary concentrator (EYELA N-1200B) at a vacuum of 110 mmHg and an external temperature of 70°C. Hydrochloric acid was added to achieve concentrations of 4, 5, 6, and 6.5 N, respectively. After 1 hour at 60°C, the absorbance changes of the concentrates were measured.

Each L-cysteine concentrate (prepared solution) comprising various concentrations of hydrochloric acid was cooled to 15°C in a jacket tank with stirring at a constant cooling rate for 4 hours. Then, L-cysteine crystals were separated from the L-cysteine crystal slurry using a basket separator at a rotation speed of 3,000 rpm for 15 minutes. After separation, drying was performed in an oven dryer at 40°C for more than 2 hours to reduce the residual moisture to 0.5% or less, and finally L-Cysteine hydrochloride crystals were manufactured. The yellowness (b* value) of the manufactured L-Cysteine hydrochloride crystals was measured using a colorimeter of Chroma meter (CR-410, KONICA MINOLTA), and the results are shown in Table 1 below. The closer the yellowness (b* value) is to 0, the closer it is to white, and the higher the value, the more yellow it is.

**[Table 1]**

| Concentration of HCl (N) | Initial Absorbance (430 nm) | Final Absorbance (430 nm) | b* value of crystal |
|---|---|---|---|
| 4 | 0.02 | 0.13 | 2.1 |
| 5 | 0.02 | 0.35 | 2.5 |
| 6 | 0.02 | 0.55 | 4 |
| 6.5 | 0.02 | 0.65 | 7.1 |

As a result, as shown in Table 1, it could be seen that the increase in absorbance value was greater as the hydrochloric acid concentration of the concentrate solution (preparation solution) increased. In addition, it was confirmed that the color value of L-cysteine hydrochloride crystals was determined according to the final absorbance value of the concentrate solution (preparation solution). It was confirmed that when the absorbance value was 0.55 or less, L-cysteine hydrochloride crystals having a yellowness (b* value) of 4.0 or less could be obtained.

### Example 2. Comparison of absorbance of fermentation-derived concentrates containing L-cysteine according to residence temperature

The following experiment was conducted to determine the effect of residence temperature on the absorbance of fermentation-derived concentrates containing L-cysteine.

Specifically, 5 wt% activated carbon was added to the L-cysteine-separated solution obtained using the continuous chromatography process of Example 1-1, and the mixture was stirred at 40°C for 1 hour. The activated carbon was separated using a Nutsche filter to obtain a filtrate. The filtrate was concentrated in a rotary evaporator at a vacuum of 110 mmHg and an external temperature of 70°C. Hydrochloric acid was then added to produce concentrates with concentrations of 4.5, 5.5, and 6.5 N. The residence temperatures of each L-cysteine concentrate containing various concentrations of hydrochloric acid were set to 20°C, 40°C, and 60°C, respectively, and the solutions were allowed to remain for 1 hour, 2 hours, 3 hours, 4 hours, 6 hours, and 8 hours, and then the UV/Vis absorbance at 430 nm was measured to test the change in absorbance over time. The results of the absorbance measurement over time are shown in Table 2 below.

**[Table 2]**

| Residence temperature (°C) | Concen tration of HCl (N) | Absorbance (430 nm) depending on the residence time | | | | | | | Change rate |
|---|---|---|---|---|---|---|---|---|---|
| | | 0 hr | 1 hr | 2 hr | 3 hr | 4 hr | 6 hr | 8 hr | Absorbance /hr |
| 20 | 4.5 | 0.15 | 0.15 | 0.15 | 0.16 | 0.16 | 0.17 | 0.17 | 0.003 |
| | 5.5 | 0.14 | 0.15 | 0.15 | 0.15 | 0.15 | 0.17 | 0.17 | 0.004 |
| | 6.5 | 0.15 | 0.15 | 0.15 | 0.16 | 0.16 | 0.17 | 0.18 | 0.004 |
| 40 | 4.5 | 0.15 | 0.19 | 0.21 | 0.24 | 0.30 | 0.36 | 0.43 | 0.04 |
| | 5.5 | 0.14 | 0.18 | 0.22 | 0.30 | 0.35 | 0.50 | 0.62 | 0.06 |
| | 6.5 | 0.15 | 0.29 | 0.35 | 0.45 | 0.68 | 0.95 | 1.21 | 0.13 |
| 60 | 4.5 | 0.15 | 0.28 | 0.37 | 0.41 | 0.61 | 0.86 | 1.11 | 0.12 |
| | 5.0 | 0.15 | 0.27 | 0.45 | 0.62 | 0.91 | 1.26 | 1.54 | 0.17 |
| | 5.5 | 0.14 | 0.36 | 0.74 | 0.78 | 1.13 | 1.74 | 2.34 | 0.28 |
| | 6.0 | 0.14 | 0.44 | 0.91 | 1.03 | 1.58 | 2.27 | 2.87 | 0.34 |
| | 6.5 | 0.15 | 0.47 | 1.11 | 1.38 | 2.07 | 2.69 | 3.63 | 0.43 |

As a result shown in Table 2, it was confirmed that the higher the residence temperature of the concentrate and the hydrochloric acid concentration, the greater the increase in absorbance. Unlike the results of Examples 1-2 above, when maintained (stored) at 20°C, the change in absorbance per hour hardly occurred at 0.003-0.004 even when the hydrochloric acid concentration and the residence time increased. It was confirmed that when the hydrochloric acid concentration increased from 4.5 N to 6.5 N At 40°C, the change in absorbance per hour increased linearly from 0.04 to 0.13, and when the hydrochloric acid concentration increased from 4.5 N to 6.5 N at 60°C, the change in absorbance per hour increased linearly from 0.12 to 0.43. Therefore, it was confirmed that the change in absorbance of the concentrate was affected by a specific substance derived from the fermentation solution depending on the conditions of residence temperature and hydrochloric acid concentration.

### Comparative Example 1. Effect of input amount of activated carbon on removal of color-inducing substances

In addition to the concentration of hydrochloric acid added to the concentrate and the residence temperature of the concentrate, which were conditions for controlling the color of L-cysteine hydrochloride crystals derived from Examples 1 and 2, the following experiment was conducted to determine the effect of input amount of activated carbon on the removal of color-inducing substances.

Specifically, activated carbon was added to the L-cysteine separated solution obtained by continuous chromatography at concentrations of 0, 5, and 10 wt% based on the total weight of L-cysteine, and the mixture was stirred at 40°C for 1 hour. The activated carbon was separated using a Nutsche filter to obtain a filtrate, which was then concentrated in a rotary evaporator at a vacuum of 110 mmHg and an external temperature of 70°C. After concentration, hydrochloric acid was added to make a concentrate so that the hydrochloric acid concentration became 5.5 N, and the results of measuring the change in absorbance of the concentrate over time at 60°C every 2 hours are shown in Table 3 below.

**[Table 3]**

| Activated carbon | Absorbance (430 nm) depending on the residence time | | | | Change rate |
|---|---|---|---|---|---|
| wt% | 0 hr | 2 hr | 4 hr | 8 hr | Absorbance /hr |
| 0 | 0.34 | 0.97 | 1.85 | 3.12 | 0.35 |
| 5 | 0.14 | 0.74 | 1.13 | 2.34 | 0.28 |
| 10 | 0.02 | 0.65 | 0.94 | 2.04 | 0.25 |

As a result shown in Table 3, the increase in absorbance per hour of the concentrate after decolorization by adding activated carbon at an amount of 10 wt% was confirmed to be at a similar level of 0.25 to 0.35. Therefore, although a small amount of color-inducing substances could be removed by the decolorization process using activated carbon, the effect was not great. Thus, it was confirmed that the conditions of the concentration of hydrochloric acid added to the concentrate derived from Examples 1 and 2 and the residence temperature of the concentrate were the most important factors for controlling the color intensity of L-cysteine hydrochloride crystals.

### Experimental Example 1. Effect of L-cysteine reagent on absorbance change according to PLP concentration

Pyridoxal 5'-phosphate (PLP) is a coenzyme used in a method (US 8557549 B2) for producing cysteine or its derivatives by reacting O-phosphoserine (OPS) as a substrate with sulfide in the presence of O-phosphoserine sulfhydrylase (OPSS) or a microorganism expressing OPSS. The following experiment was conducted to determine the effect of the concentration of the coenzyme PLP (pyridoxal-5'-phosphate) on the change in color intensity of an L-cysteine solution.

Specifically, 6.0 N hydrochloric acid was added to 100 g/L of L-cysteine reagent solutions which were not added with PLP, or added with PLP at concentrations of 1 wt%, 5 wt%, and 10 wt%, respectively. Then, the change in UV/V is absorbance at 430 nm of each sample solution was measured every 2 hours at a residence temperature of 60°C, and the results are shown in Fig. 1 and Table 4 below.

**[Table 4]**

| PLP concentration | Absorbance (430 nm) | | |
|---|---|---|---|
| wt% | 0 hr | 2 hr | 4 hr |
| 0 | 0 | 0 | 0 |
| 1 | 0 | 0.13 | 0.02 |
| 5 | 0 | 0.16 | 0.28 |
| 10 | 0 | 0.56 | 0.98 |

As a result, as shown in Fig. 1 and Table 4, under conditions of no addition of PLP, the absorbance of the L-cysteine sample solution did not change over residence time. However, under conditions with addition of PLP, the rate of change in absorbance of the L-cysteine sample solution over time increased as the PLP concentration increased. Furthermore, at a residence temperature of 60°C, the color of the L-cysteine solution changed from yellow to orange as the residence time increased (as the heating time increased) (see Fig. 1).

These results confirmed that PLP might be included in the fermentation broth-derived substance that affected absorbance depending on the conditions of the residence temperature and hydrochloric acid concentration in Example 2.

## Claims

1. A method for preparing L-cysteine crystals, comprising steps of:
(a) obtaining an L-cysteine separated solution from a fermentation broth comprising L-cysteine;
(b) filtering the L-cysteine separated solution to obtain a filtrate;
(c) concentrating the filtrate to obtain a concentrate;
(d) adding hydrochloric acid to the filtrate or the concentrate, before or after step (c); and
(e) cooling the concentrate to recover L-cysteine crystals.

2. The method for producing L-cysteine crystals according to claim 1, wherein the step of obtaining an L-cysteine separated solution in step (a) is performed using continuous chromatography.

3. The method for preparing L-cysteine crystals according to claim 1, wherein the step of obtaining a filtrate in step (b) is performed by adding activated carbon.

4. The method for preparing L-cysteine crystals according to claim 3, wherein the activated carbon is added in an amount of 0.01 to 50 wt% based on the total weight of L-cysteine.

5. The method for preparing L-cysteine crystals according to claim 1, wherein the concentrating in step (c) is carried out under conditions of a vacuum degree of 100 to 120 mmHg and an external temperature of 60 to 80°C.

6. The method for preparing L-cysteine crystals according to claim 1, wherein the amount of hydrochloric acid added in step (d) is such that the hydrochloric acid concentration of the concentrate after addition is less than 6.5 N.

7. The method for preparing L-cysteine crystals according to claim 1, further comprising a step of controlling a residence time of the concentrate at a temperature of 10°C to 70°C after step (d).

8. The method for preparing L-cysteine crystals according to claim 7, wherein the residence time at 10°C or higher and less than 30°C of a residence time is within 24 hours, when the hydrochloric acid concentration of the concentrate after addition of hydrochloric acid in step (d) is 4.5 N or higher.

9. The method for preparing L-cysteine crystals according to claim 7, wherein the residence time at 30°C or higher and less than 50°C of the residence temperature is:
(i) within 720 minutes, when the hydrochloric acid concentration of the concentrate after the addition of hydrochloric acid in step (d) is less than 4.5 N;
(ii) within 480 minutes, when the hydrochloric acid concentration of the concentrate after the addition of hydrochloric acid in step (d) is between 4.5 N or higher and less than 5.5 N; or
(iii) within 240 minutes, when the hydrochloric acid concentration of the concentrate after the addition of hydrochloric acid in step (d) is between 5.5 N or higher and less than 6.5 N.

10. The method for preparing L-cysteine crystals according to claim 7, wherein the residence time at 50°C or higher of the residence temperature is:
(i) within 240 minutes, when the hydrochloric acid concentration of the concentrate after the addition of hydrochloric acid in step (d) is less than 4.5 N;
(ii) within 180 minutes, when the hydrochloric acid concentration of the concentrate after the addition of hydrochloric acid in step (d) is between 4.5 N or higher and less than 5.5 N; or
(iii) within 120 minutes, when the hydrochloric acid concentration of the concentrate after the addition of hydrochloric acid in step (d) is between 5.5 N or higher and less than 6.5 N.

11. The method for preparing L-cysteine crystals according to claim 7, wherein an absorbance of the concentrate at 430 nm after adjusting the residence time is 0.55 or less.

12. The method for preparing L-cysteine crystals according to claim 1, wherein the concentrate in step (e) is cooled to a temperature of 0 to 30°C.

13. The method for preparing L-cysteine crystals according to any one of claims 1 to 12, wherein the L-cysteine crystals have a yellowness of 4.0 or less.
